Europäisches Patentamt

European Patent Office

Office européen des brevets

(11) Numéro de publication : **0 440 618 B1**

(12) **FASCICULE DE BREVET EUROPEEN**

(45) Date de publication du fascicule du brevet :
**07.10.92 Bulletin 92/41**

(21) Numéro de dépôt : **89903184.3**

(22) Date de dépôt : **02.03.89**

(86) Numéro de dépôt international :
**PCT/FR89/00083**

(87) Numéro de publication internationale :
**WO 89/07916 08.09.89 Gazette 89/21**

(51) Int. Cl.⁵ : **A61F 2/04,** A61B 19/00

(54) **ENDOPROTHESE TUBULAIRE POUR CONDUITS ANATOMIQUES, ET INSTRUMENT POUR SA MISE EN PLACE.**

(30) Priorité : **02.03.88 FR 8802835**

(43) Date de publication de la demande :
**14.08.91 Bulletin 91/33**

(45) Mention de la délivrance du brevet :
**07.10.92 Bulletin 92/41**

(84) Etats contractants désignés :
**AT BE CH DE FR GB IT LI LU NL SE**

(56) Documents cités :
**EP-A- 0 146 794**
**FR-A- 1 103 165**
**FR-A- 2 122 032**
**FR-A- 2 248 015**
**FR-A- 2 391 709**
**US-A- 3 818 515**
**US-A- 4 592 341**
**US-A- 4 699 611**
**US-A- 4 728 328**

(56) Documents cités :
**American Society for Artificial Internal Organs, vol. 31, 1er-3 mai 1985, "Transactions", (Atlanta, Georgia, US), S. Westaby:"Long-term intubation with silicone rubber endotracheal prostheses for injury or inoperable major airways obstruction", pages318-323**

(73) Titulaire : **ARTEMIS**
**9, rue Lafon**
**F-13292 Marseille Cedex 06 (FR)**
Titulaire : **OFFICE MEDITERRANEEN DE DISTRIBUTION PHARMACEUTIQUE O.M.D.P. DEPARTEMENT COMETH**
**38, Boulevard Gay-Lussac**
**F-13014 Marseille (FR)**
Titulaire : **DUMON, Jean-Francois**
**Clos d'Albizzi 3, avenue de la Gare**
**F-13260 Cassis (FR)**

(72) Inventeur : **DUMON, Jean-François**
**Clos d'Albizzi**
**3, avenue de la Gare F-13260 Cassis (FR)**

(74) Mandataire : **Marek, Pierre**
**28 & 32 rue de la Loge**
**F-13002 Marseille (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 440 618 B1

## Description

La présente invention concerne une endoprothèse tubulaire pour conduits ou canaux anatomiques. Selon un exemple d'application particulièrement intéressante, quoique nullement limitative, cette endoprothèse est destinée à permettre la désobstruction des conduits atteints de sténose tels que trachée ou bronches, ou à servir de tuteur à de tels conduits anatomiques, lorsqu'ils s'avèrent défaillants.

On connait (FR-A-1.103.165) une canule pour le traitement de la sténose de l'oesophage, constituée par un tube flexible comportant une surface externe cannelée et destinée à être installée dans le rétrécissement tumoral pour permettre le passage des aliments.

Cette canule a pour inconvénient de n'offrir aucune garantie de maintien en place durable, en raison du fait qu'elle peut facilement tourner sur elle-même, ce qui peut provoquer une irritation génératrice de spasmes de rejet favorisant son déplacement axial, avec les graves conséquences que cela peut entraîner.

On connait aussi (US-A-4592341) d'autres instruments tubulaires ayant une pluralité des aspérités ou indentations pointues formées sur la periphérie exterieure pour éviter un retrait involontaire.

La présente invention a notamment pour but de remédier effectivement à cette sérieuse insuffisance des endoprothèses connues.

Selon l'invention, cet objectif est atteint au moyen d'une endoprothèse comportant un corps tubulaire dont la surface extérieure est munie d'une pluralité de protubérances ou aspérités de préférence réparties sur la totalité ou la quasi totalité de ladite surface, ces protubérances étant constituées par des tétons à sommet arrondis espacés les uns des autres dans le sens longitudinal et dans le sens périphérique dudit corps tubulaire, ces tétons étant, de préférence, orientés radialement.

La prothèse selon l'invention présente l'avantage de pouvoir être mise en place facilement et de se fixer d'une façon naturelle, extrêmement résistante, à son emplacement d'implantation, ladite prothèse ne pouvant, en effet, ni tourner, ni glisser axialement lorsqu'elle est installée dans la sténose. En outre, cette prothèse réalisée dans une matière plastique telle qu'un silicone élastomère est bien tolérée par l'organisme et les malades chez qui elle ne produit ni spasmes de rejet, ni traumatisme.

Les buts, caractéristiques et avantages ci-dessus et d'autres encore, ressortiront mieux de la description qui suit et des dessins annexés dans lesquels :

La figure 1 est une vue en perspective d'un exemple d'exécution de l'endoprothèse tubulaire selon l'invention.

La figure 2 est une vue en coupe longitudinale de cette endoprothèse.

La figure 3 en est une vue en coupe transversale.

La figure 4 est une vue de détail, à plus grande échelle et en coupe longitudinale de l'une des extrémités de l'endoprothèse.

La figure 5 est une vue de face d'une variante d'exécution de l'endoprothèse selon l'invention, par exemple destinée à permettre une désobstruction trachéobronchique.

La figure 6 est une vue de l'instrument permettant la mise en place d'une endoprothèse du genre de celle qui est illustrée aux figures 1 à 4.

Les figures 7 à 11 illustrent la mise en oeuvre de cet instrument et la méthode de mise en place de l'endoprothèse tubulaire selon l'invention.

La figure 12 est une vue de face d'un autre mode d'exécution d'une endoprothèse trachéobronchique selon l'invention.

On se réfère auxdits dessins pour décrire des exemples intéressants, mais non limitatifs, de réalisation de cette endoprothèse, ainsi qu'un exemple d'exécution de l'instrument permettant sa mise en place.

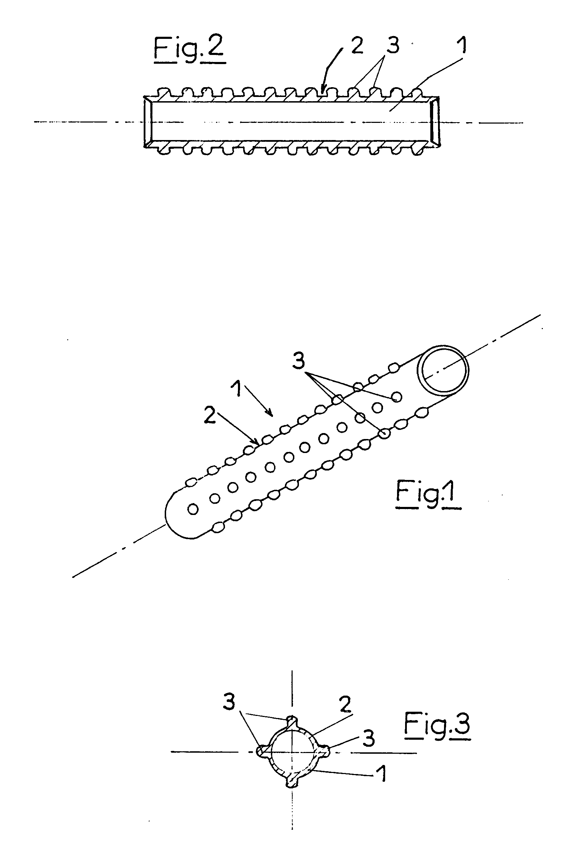

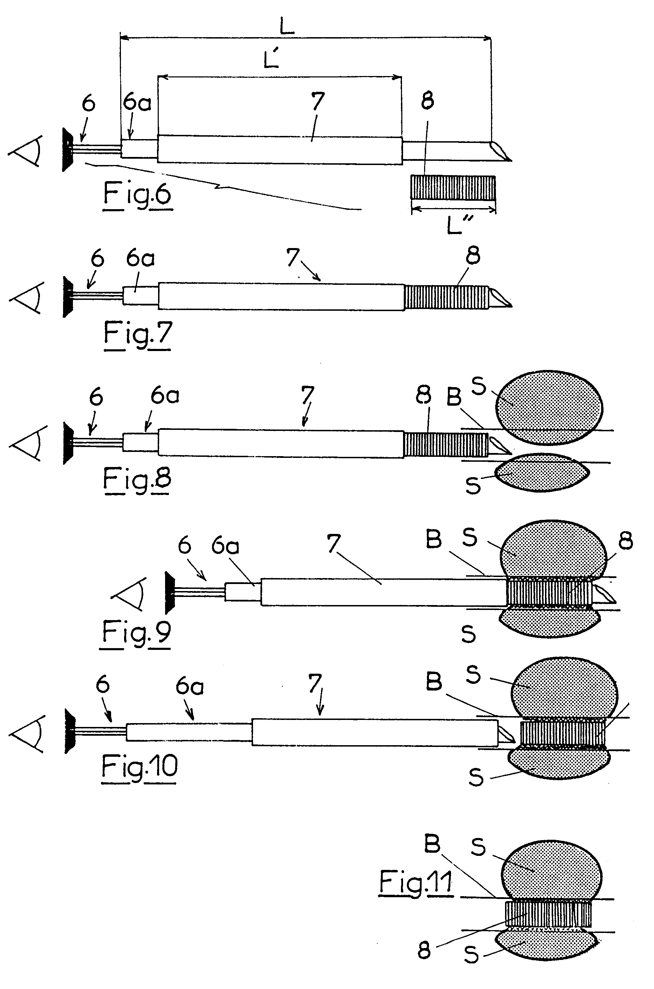

L'endoprothèse tubulaire selon l'invention peut, en fonction de la conformation du conduit ou canal anatomique à l'intérieur duquel elle est destinée à être installée, affecter une forme simple, rectiligne ou sensiblement rectiligne (par exemple lorsqu'il s'agit d'endoprothèse trachéale ou d'endoprothèse bronchique), comme le montre la figure 1, ou une forme courbe, ou une forme plus complexe comprenant un tube principal se prolongeant par deux branches tubulaires divergentes (lorsqu'il s'agit d'endoprothèse trachéobronchique), comme illustré à la figure 5. Plus précisément, cette endoprothèse peut avoir toute forme et tout diamètre adaptés à la forme et au diamètre des conduits, canaux ou vaisseaux à l'intérieur desquels elle est appelée à être placée. Elle peut être réalisée en tous matériaux souples, semi-rigides ou rigides, renforcés ou non par une armature interne, susceptibles d'être bien tôlérés par l'organisme. Elle peut, par exemple, être avantageusement exécutée dans un matériau doté d'une capacité de déformation élastique tel que, de manière intéressante, un silicone élastomère.

Selon l'invention, cette endoprothèse comporte un corps tubulaire 1 et elle est principalement remarquable par le fait que sa surface extérieure 2 destinée à se trouver au contact de la paroi interne des conduits anatomiques, est munie d'une pluralité de protubérances ou aspérités 3, de préférence régulièrement réparties sur la totalité ou la quasi totalité de ladite surface.

Ces protubérances ou aspérités 3 peuvent avoir des formes très diverses, dépourvues d'arêtes susceptibles de blesser les parois anatomiques avec lesquelles elles sont destinées à se trouver en contact. Elles sont constituées par des tétons à sommet arrondi, disposés en alignements orientés suivant des génératrices de l'endoprothèse ; ces alignements de têtons étant angulairement espacés, comme le montre notamment la figure 1. Ces têtons 3 sont orientés radialement par rapport au corps tubulaires 1 et ils sont espacés les uns des autres, aussi bien dans la direction longitudinale que dans la direction périphérique. Ils peuvent être avantageusement distribués en quinconce sur la surface latérale du corps tubulaire 1. Ils peuvent avoir une section circulaire ou toute autre section. En outre, ces têtons ou ergots 3 sont ineffaçables, c'est-à-dire qu'ils sont suffisamment rigides pour ne pas être comprimés, pliés ou escamotés, dans les conditions d'utilisation.

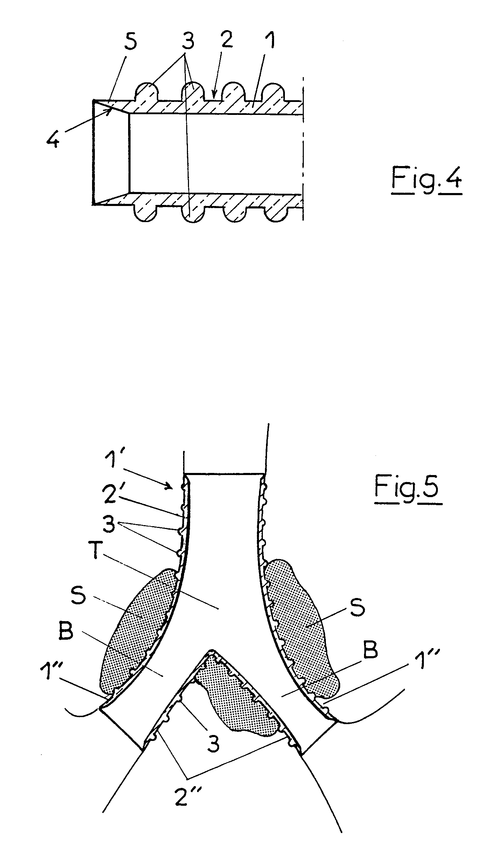

L'une au moins et, de préférence, les deux extrémités de l'endoprothèse sont dotées d'un biseau interne 4 (figure 4), de sorte que lesdites extrémités sont constituées par des lèvres minces et effilées qui, lorsque ladite endoprothèse se trouve en place, ne forment aucune aspérité rétentrice favorisant la formation d'amas de nature diverse, en fonction de la nature des conduits, canaux ou vaisseaux, à l'entrée ou à la sortie de la prothèse.

La variante d'exécution de la prothèse illustrée à la figure 5 diffère de la précédente seulement par sa forme un peu plus complexe adaptée à la conformation des conduits dans lesquels elle est appelée à être installée. Selon cette variante de réalisation, la prothèse comprend un corps tubulaire principal 1' se prolongeant par deux branches tubulaires divergeantes 1" ; les surfaces extérieures 2', 2" de ce corps principal et de ces branches étant munies de têtons 3, comme indiqué précédemment. Une telle prothèse est destinée à être placée au point de ramification d'un conduit anatomique. Selon une application très intéressante, une telle prothèse est destinée à constituer une endoprothèse trachéobronchique permettant de "court-circuiter" une obstruction S affectant à la fois la partie inférieure de la trachée T et la portion initiale des deux bronches B.

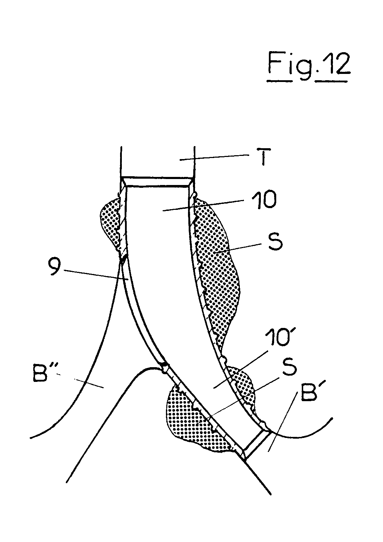

La figure 12 montre une autre forme d'exécution d'une prothèse, telle qu'une endoprothèse trachéobronchique, également destinée à être placée au point de ramification d'un conduit anatomique, par exemple dans les cas où l'obstruction S à courtcircuiter affecte seulement la base du conduit principal (trachée T, par exemple) et l'une seulement des ramifications (l'une des bronches B', par exemple). Dans ce cas, la prothèse présente une conformation courbe et comprend une partie principale 10 se prolongeant par une partie secondaire de diamètre plus réduit 10'. Elle comporte, en outre, latéralement, une ouverture 9 disposée au point de jonction de ses parties principales 10 et secondaire 10' et destinée à être placée à l'entrée de la seconde ramification saine (l'autre bronche B", par exemple), de façon à permettre une communication entre ledit conduit principal T et ladite ramification saine B".

L'ouverture latérale 9 peut également permettre et favoriser la mise en place d'une deuxième branche tubulaire indépendante semblable à la partie secondaire 10', de façon à réaliser une endoprothèse du genre de celle qui est illustrée à la figure 5.

On a illustré, à la figure 6, un instrument de mise en place ou introducteur des endoprothèses constituées par un tube rectiligne ou sensiblement rectiligne telles que celles qui sont destinées à être installées dans la trachée ou dans les bronches défaillantes ou atteintes de sténose ou de tumeur compressive, afin de leur servir de tuteur ou de permettre leur désobstruction.

Cet instrument comprend un bronchoscope classique 6 sur le corps cylindrique allongé ou tige de guidage 6a duquel est monté, avec une aptitude de coulissement par rapport à ladite tige, un poussoir tubulaire 7 dont la longueur L' est inférieure à la longueur L de cette dernière. La différence de longueur entre la tige 6a du bronchoscope et le poussoir 7 correspond à au moins la longueur L" de l'endoprothèse 8 destinée à être introduite et positionnée à l'aide de l'instrument ; cette différence de longueur (L - L') étant, cependant, de préférence supérieure à la longueur L" de ladite endoprothèse.

Les figures 7 à 11 illustrent le procédé de mise en place d'une endoprothèse simple 8 dans un conduit anatomique B (par exemple trachée ou bronche) obstrué par une tumeur compressive S.

La prothèse 8 est d'abord placée autour de l'extrémité de la tige 6a du broncoscope 6, en avant du poussoir 7 (figure 7), l'extrémité effilée de la tige émergeant à l'avant de ladite prothèse, afin de faciliter la progression dans le conduit anatomique.

La tige munie de la prothèse 8 est ensuite introduite et enfoncée dans le conduit anatomique B (figure 8) jusqu'à ce que ladite prothèse soit amenée à l'emplacement souhaitable (figure 9), c'est-à-dire à l'emplacement où se trouve la sténose ou tumeur compressive.

On retire alors la tige 6a du bronchoscope en maintenant en place le poussoir 7 qui empêche tout mouvement de recul de la prothèse durant ce retrait (figure 10). On retire enfin le poussoir, la prothèse se trouvant alors installée et solidement fixée dans son logement grâce aux têtons 3 dont est pourvue sa surface extérieure sur laquelle la paroi anatomique environnante exerce une pression empêchant tout déplacement et mouvement de la prothèse.

## Revendications

1. - Endoprothèse tubulaire pour conduits anatomiques, comportant un corps tubulaire 1, dont la surface extérieure (2) est munie d'une pluralité de protubérances ou aspérités (3), de préférence réparties sur la totalité de ladite surface extérieure (2) caractérisée en ce que lesdites protubérances ou aspérités (3) sont constituées par des têtons à sommet arrondi espacés les uns des autres dans le sens longitudinal et dans le sens périphérique dudit corps tubulaire (1).

2. - Endoprothèse tubulaire selon la revendication 1, caractérisée en ce que les têtons (3) sont orientés radialement par rapport au corps tubulaire (1).

3. - Endoprothèse tubulaire selon l'une des revendications 1 ou 2, caractérisée en ce que les têtons (3) sont disposés en alignements espacés et orientés suivant des génératrices du corps tubulaire (1) de ladite endoprothèse.

4. - Endoprothèse tubulaire selon l'une des revendications 1 ou 2, caractérisée en ce que les têtons (3) sont répartis en quinconce sur la surface extérieure (2) du corps tubulaire (1).

5. - Endoprothèse tubulaire suivant la revendication 1, caractérisée en ce que l'une au moins de ses extrémités comporte un biseau interne (4).

6. - Endoprothèse tubulaire selon la revendication 1, caractérisée en ce que ses deux extrémités ou toutes ses extrémités sont dotées d'un biseau interne (4).

7. - Endoprothèse tubulaire suivant l'une des revendications 1 ou 2, caractérisée en ce qu'elle est exécutée dans un matériau doté d'une capacité de déformation élastique, tel que, par exemple, un silicone élastomère.

8. - Endoprothèse tubulaire suivant l'une des revendications 1 ou 2, caractérisée en ce qu'elle comporte, latéralement, une ouverture (9) destinée à conserver une communication entre le conduit (T-B') dans lequel est logée ladite endoprothèse et une ramification (B").

**Patentansprüche**

1. Röhrenförmige Endoprothese für anatomische Gänge oder Kanäle, bestehend aus einem rohrförmigen Körper (1), dessen Außenfläche (2) mit einer Vielzahl von Vorsprüngen oder Unebenheiten (3) versehen ist, die vorzugsweise über die gesamte Außenfläche (2) verteilt sind, dadurch gekennzeichnet, daß die Protuberanzen oder Unebenheiten (3) als Zapfen mit abgerundeter Spitze ausgebildet sind, die voneinander in Längsrichtung und in Umfangsrichtung des rohrförmigen Körpers (1) beabstandet sind.

2. Röhrenförmige Endoprothese nach Anspruch 1, dadurch gekennzeichnet, daß die Zapfen (3) bezüglich des rohrförmigen Körpers (1) radial ausgerichtet sind.

3. Röhrenförmige Endoprothese nach einem der Ansprüche 1 oder 2, dadurch gekennzeichnet, daß die Zapfen (3) in voneinander beabstandeten Reihen angeordnet sind, die entsprechend den Mantellinien des rohrförmigen Körpers (1) der Endoprothese ausgerichtet sind.

4. Röhrenförmige Endoprothese nach einem der Ansprüche 1 oder 2, dadurch gekennzeichnet, daß die Zapfen (3) versetzt auf der Außenfläche (2) des rohrförmigen Körpers (1) angeordnet sind.

5. Röhrenförmige Endoprothese nach Anspruch 1, dadurch gekennzeichnet, daß mindestens ein Ende eine innere Abschrägung aufweist (4).

6. Röhrenförmige Endoprothese nach Anspruch 1, dadurch gekennzeichnet, daß ihre beiden Enden oder alle Enden mit einer inneren Abschrägung (4) versehen sind.

7. Röhrenförmige Endoprothese nach einem der Ansprüche 1 oder 2, dadurch gekennzeichnet, daß sie aus einem elastisch verformbaren Material besteht, wie zum Beispiel einem elastomeren Silikon.

8. Röhrenförmige Endoprothese nach einem der Ansprüche 1 oder 2, dadurch gekennzeichnet, daß sie seitlich eine Öffnung (9) aufweist, die dazu bestimmt ist, eine Verbindung zwischen dem Gang (T-B'), in dem die Endoprothese eingesetzt ist, und einer Verzweigung (B") zu gewährleisten.

**Claims**

1. Tubular endoprosthesis for anatomical passages, comprising a tubular body (1), the external surface (2) of which is provided with a plurality of protuberances or bumps (3), preferably distributed over the whole of said external surface (2), characterised in that said protuberances or bumps (3) are constituted by knobs with rounded tops spaced apart longitudinally and peripherally over said tubular body (1).

2. Tubular endoprosthesis according to claim 1, characterised in that the knobs (3) are radially orientated in relation to the tubular body (1).

3. Tubular endoprosthesis according to either claim 1 or 2, characterised in that the knobs (3) are arranged in lines spaced apart and orientated in generating lines on the tubular body (1) of said endoprosthesis.

4. Tubular endoprosthesis according to either claim 1 or 2, characterised in that the knobs (3) are staggered over the external surface (2) of the tubular body (1).

5. Tubular endoprosthesis according to claim 1, characterised in that at least one of its ends is bevelled internally (4).

6. Tubular endoprosthesis according to claim 1, characterised in that both its ends or all of its ends are bevelled internally (4).

7. Tubular endoprosthesis according to either claim 1 or 2, characterised in that it is made in a material having the ability to be elastically deformed, for example a silicone elastomer.

8. Tubular endoprosthesis according to either claim 1 or 2, characterised in that it comprises, laterally, an opening (9) designed to maintain communication between the passage (T-B′) in which said endoprosthesis is accommodated and a branch (B″).

Fig.2

Fig.1

Fig.3

Fig.4

Fig.5

Fig.6

Fig.7

Fig.8

Fig.9

Fig.10

Fig.11

Fig.12